(19) Europäisches Patentamt / European Patent Office / Office européen des brevets

(11) **EP 4 068 296 A1**

(12) **DEMANDE DE BREVET EUROPEEN**

(43) Date de publication:
**05.10.2022 Bulletin 2022/40**

(21) Numéro de dépôt: **22166010.3**

(22) Date de dépôt: **31.03.2022**

(51) Classification Internationale des Brevets (IPC):
**G16H 20/13** *(2018.01)*    **G16H 40/63** *(2018.01)*

(52) Classification Coopérative des Brevets (CPC):
**G16H 20/13; G16H 40/63**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **01.04.2021 FR 2103413**

(71) Demandeurs:
• **Hephaï**
  **75011 Paris (FR)**
• **Assistance Publique-Hôpitaux de Paris (AP-HP)**
  **75004 Paris (FR)**
• **Sorbonne Université**
  **75006 Paris 6 (FR)**

• **INSTITUT NATIONAL DE LA SANTE ET DE LA RECHERCHE MEDICALE - INSERM**
  **75013 Paris (FR)**

(72) Inventeurs:
• **BELANGER-BASSET, Geneviève, Isabelle**
  **92240 MALAKOFF (FR)**
• **MINVIELLE, Ludovic, Louis, Jean-Pierre**
  **33800 BORDEAUX (FR)**
• **TROSINI DESERT, Valéry**
  **75011 PARIS (FR)**
• **SIMILOWSKI, Thomas**
  **92130 ISSY LES MOULINEAUX (FR)**

(74) Mandataire: **Santarelli**
  **49, avenue des Champs-Elysées**
  **75008 Paris (FR)**

(54) **DETECTION DE LA SYNCHRONISATION ENTRE L'ACTIONNEMENT D'UN INHALATEUR-DOSEUR SOUS PRESSION ET L'INSPIRATION D'UN PATIENT**

(57)    Un inhalateur-doseur sous pression (300) requiert une bonne synchronisation entre l'activation de l'inhalateur et l'inspiration du patient. Des traitements sont réalisés sur des trames vidéos filmant le patient pour qualifier l'actionnement de l'inhalateur-doseur sous pression selon deux critères : au regard de l'appui des doigts actuateur du patient sur un élément déclencheur de l'inhalateur et au regard de la compression propre de l'inhalateur. Un traitement d'un signal audio enregistrant en même temps le patient est également réalisé pour détecter une inhalation du patient. Une corrélation temporelle de probabilités obtenues en résultats permet alors de qualifier la synchronisation entre l'actionnement de l'inhalateur par le patient et une inspiration de celui-ci, et ainsi remonter un bon usage ou un mésusage de l'inhalateur.

Figure 2

EP 4 068 296 A1

## Description

### Domaine technique

L'invention concerne le suivi de l'usage ou utilisation d'un inhalateur doseur pressurisé par un patient soumis un traitement thérapeutique inhalé, typiquement médicamenteux.

### Techniques antérieures

La pierre angulaire du traitement de l'asthme et de la bronchopneumopathie chronique obstructive, BPCO, repose sur des inhalateurs prêts à l'emploi prescrits au long cours.

Une bonne utilisation des dispositifs d'inhalation est cruciale au soulagement des symptômes de l'asthme et de la BPCO et à la prévention des exacerbations de ces maladies. Une bonne observance de la prise de l'inhalateur et un bon usage de l'inhalateur sont deux éléments fondamentaux pour une bonne efficacité du traitement.

30 à 40% des patients ne savent pas utiliser correctement leur inhalateur. C'est ce qu'on appelle le mésusage. Ce dernier a des conséquences médicales et économiques non négligeables. Il est donc combattu.

Le document US 2013/063579 décrit un système de détection du bon actionnement d'un inhalateur combinant des traitements vidéo et audio. La vidéo est traitée pour vérifier le positionnement du visage de l'utilisateur-patient, le positionnement correct du dispositif d'inhalation, puis l'actionnement de l'inhalateur. Cet actionnement est confirmé à l'aide d'une analyse d'un signal audio enregistré, dans lequel un son cible est recherché. Un système de reconnaissance audio peut aussi être utilisé, lequel est entraîné pour classifier différents sons, par exemple des sons d'inhalation avec ou sans dent perturbant le flux d'air, éventuellement selon le volume d'air aspiré.

On connaît également du document WO 2019/122315 un système et un procédé qui utilisent un réseau de neurones appliqué à des signaux vidéo et audio, pour détecter le type d'inhalateur à aérosol et tout écart dans son utilisation, incluant la posture du patient, le positionnement de l'inhalateur ou encore la respiration du patient telle qu'une mauvaise synchronisation de l'actionnement de l'inhalateur.

La synchronisation entre l'actionnement de l'inhalateur à aérosol et l'inspiration du patient est cruciale pour la bonne prise médicamenteuse. Elle est en particulier délicate à réaliser et ainsi à vérifier pour des inhalateurs-doseurs sous pression. Les techniques automatisées connues ne permettent pas de détecter le mésusage résultant d'une désynchronisation de façon aussi précise que le professionnel médical observant le patient.

Il est donc un besoin d'améliorer ces techniques pour permettre une meilleure détection du mésusage des inhalateurs-doseurs sous pression de façon autonome et ainsi mieux éduquer les patients à une bonne prise médicamenteuse, en limitant l'intervention du corps médical.

### Exposé de l'invention

L'invention propose ainsi un procédé mis en œuvre par ordinateur de suivi de l'usage, par un patient, d'un inhalateur-doseur sous pression, comprenant les étapes suivantes :

obtenir un signal vidéo et un signal audio d'un patient utilisant un inhalateur-doseur sous pression,
calculer, pour chacune d'une pluralité de trames vidéo du signal vidéo, au moins l'une parmi une probabilité, dite d'appui, qu'un doigt actuateur du patient dans la trame vidéo soit en phase d'appui sur un élément déclencheur de l'inhalateur-doseur sous pression et une probabilité, dite de compression, que l'inhalateur-doseur sous pression dans la trame vidéo soit dans un état comprimé,
calculer, pour chacun d'une pluralité de segments audio du signal audio, une probabilité, dite d'inhalation, que le patient réalise, dans le segment audio, une inspiration combinée au flux aérosol,
déterminer un degré de synchronisation entre l'actionnement de l'inhalateur-doseur sous pression et une inspiration du patient à partir des probabilités d'appui, de compression et d'inhalation correspondant à des mêmes instants temporels, et
émettre en conséquence un signal de bon usage ou de mésusage de l'inhalateur-doseur sous pression au patient.

Les inventeurs ont constaté l'efficacité, en terme de détection de la synchronisation, d'une prise en compte conjointe d'une probabilité vidéo (de détection) d'action mécanique sur l'inhalateur-doseur sous pression (via les doigts actuateurs et/ou via la compression même de l'inhalateur) et d'une probabilité audio (de détection) d'une inhalation ou inspiration du patient.

Des techniques de calcul informatique permettent de façon efficace d'obtenir de telles probabilités par traitement des signaux vidéo et audio.

Corrélativement, l'invention concerne également un système informatique comprenant un ou plusieurs processeur, par exemple un ou des processeurs CPU et/ou un ou des processeurs graphiques GPU et/ou un ou des microprocesseurs, configuré pour :

obtenir un signal vidéo et un signal audio d'un patient utilisant un inhalateur-doseur sous pression,
calculer, pour chacune d'une pluralité de trames vidéo du signal vidéo, au moins l'une parmi une probabilité, dite d'appui, qu'un doigt actuateur du patient dans la trame vidéo soit en phase d'appui sur un élément déclencheur de l'inhalateur-doseur sous pression et une probabilité, dite de compression, que l'inhalateur-doseur sous pression dans la trame vidéo soit dans un état comprimé,

calculer, pour chacun d'une pluralité de segments audio du signal audio, une probabilité, dite d'inhalation, que le patient réalise, dans le segment audio, une inspiration combinée au flux aérosol,

déterminer un degré de synchronisation entre l'actionnement de l'inhalateur-doseur sous pression et une inspiration du patient à partir des probabilités d'appui, de compression et d'inhalation correspondant à des mêmes instants temporels, et

émettre en conséquence un signal de bon usage ou de mésusage de l'inhalateur-doseur sous pression au patient.

Ce système informatique peut simplement prendre la forme d'un terminal utilisateur, tel un téléphone intelligent (smartphone), une tablette numérique, un ordinateur portable, un assistant personnel, un dispositif de divertissement (e.g. console de jeu), ou encore un dispositif fixe tel qu'un ordinateur de bureau ou plus généralement une borne interactive, par exemple disposée au domicile ou dans un espace public tel une pharmacie ou un centre médical.

Des caractéristiques optionnelles de l'invention sont définies dans les revendications dépendantes. Si ces caractéristiques sont principalement exposées ci-dessous en termes de procédé, elles peuvent être transposées en caractéristiques de système ou dispositif.

Selon un mode de réalisation, déterminer un degré de synchronisation comprend déterminer, pour chaque type de probabilité, une fenêtre temporelle de haute probabilité, et

le degré de synchronisation est fonction d'un recouvrement temporel des fenêtres temporelles ainsi déterminées pour les probabilités.

Une corrélation temporelle des probabilités déterminées est ainsi obtenue à faible coût.

Selon un autre mode de réalisation, déterminer un degré de synchronisation comprend :

combiner (e.g. linéairement), pour chacun d'une pluralité d'instants temporels, les probabilités d'appui, de compression et d'inhalation correspondant audit instant temporel en une probabilité combinée, et

déterminer, à partir des probabilités combinées, un degré de synchronisation entre l'actionnement de l'inhalateur-doseur sous pression et une inspiration du patient.

Aussi, les étapes de détection (au travers des trois probabilités) sont corrélées et unifiées en une seule fonction de détection qui peut être facilement optimisée.

Dans un mode de réalisation, le procédé comprend en outre une étape consistant à comparer les probabilités combinées à une valeur seuil de bonne synchronisation.

Dans un mode de réalisation, calculer une probabilité d'appui pour une trame vidéo comprend :

détecter, dans la trame vidéo, des points représentatifs du doigt actuateur, et

déterminer un mouvement relatif de descente du bout du doigt actuateur par rapport à une base du doigt, en comparaison d'au moins une trame vidéo temporellement précédente,

la probabilité d'appui étant fonction de l'amplitude du mouvement de descente depuis une position de départ déterminée dans une trame vidéo précédente.

La prise en compte directe de l'action de l'utilisateur offre une détection améliorée.

Selon une caractéristique, calculer une probabilité d'appui pour une trame vidéo comprend une étape consistant à comparer l'amplitude du mouvement à une dimension de l'inhalateur-doseur sous pression dans la trame vidéo. La dimension (longueur) réelle de l'inhalateur est mise à l'échelle de sa dimension dans la trame vidéo afin notamment de connaître l'amplitude maximale de mouvement possible dans la trame vidéo et ainsi déterminer le degré (et donc une probabilité) de l'appui réalisé par le patient.

Dans un mode de réalisation, calculer une probabilité de compression pour une trame vidéo comprend :

comparer une longueur de l'inhalateur-doseur sous pression dans la trame vidéo avec une longueur référence de l'inhalateur-doseur sous pression, généralement dans une trame vidéo précédente.

A nouveau, la longueur (dimension) réelle de l'inhalateur mais aussi sa course de compression théorique peuvent être mises à l'échelle de leur longueur et course dans la trame vidéo pour permettre une comparaison par exemple entre la longueur de l'inhalateur, sa longueur décompressée (de référence dans une trame précédente) et sa course maximale. Une approche linéaire permet notamment d'obtenir une probabilité (entre aucune compression et une compression maximale correspondant à la course maximale).

Dans un mode de réalisation, un segment audio correspond à une section de 1 à 5 secondes (s) du signal audio, préférablement une section de 2 à 3 s. Les segments audio sont typiquement générés avec un pas inférieur à leur durée. Aussi des segments audio se chevauchant en plus ou moins grand nombre (selon ledit pas) sont générés.

Dans un mode de réalisation, calculer une probabilité d'inhalation pour un segment audio comprend :

convertir le segment audio en un spectrogramme, et utiliser le spectrogramme en entrée d'un réseau de neurones entrainé qui émet en sortie la probabilité d'inhalation. Les inventeurs ont constaté l'efficacité d'une modélisation de spectrogrammes du signal audio dans la reconnaissance d'une inspiration du patient combinée au bruit du flux aérosol.

En variante, calculer une probabilité d'inhalation pour un segment audio comprend : calculer une distance entre un profil du segment audio et un profil de référence. Cette distance peut alors être convertie en probabilité. Un profil de segment audio peut typiquement être formé du signal audio lui-même, d'une transformée fréquentielle de celui-ci (e.g. une transformée de Fourier, rapide ou non), d'un vecteur de paramètres, notamment de paramètres MFCC pour Mel-Frequency Cepstral Coefficients.

Dans un mode de réalisation, les étapes consistant à calculer les probabilités d'appui, de compression et d'inhalation sur des trames vidéo et segments audio postérieurs sont déclenchées par la détection d'un bon positionnement de l'inhalateur-doseur sous pression par rapport au patient dans des trames vidéos antérieures. Aussi, la détermination de la bonne ou mauvaise synchronisation peut être conduite de façon automatique pour les instants temporels ultérieurs uniquement, notamment sur les trames vidéos ultérieures.

Dans un autre mode de réalisation, le procédé comprend en outre une étape préalable de détermination d'une ouverture de l'inhalateur-doseur par détection d'un son clic caractéristique dans au moins un segment audio du signal audio, la détection mettant en œuvre un modèle de détection appris. Cette détermination peut éventuellement combiner une détection via le signal vidéo. La détection de l'ouverture peut notamment constituer un événement déclencheur de la suite des détections, et notamment celle du degré de synchronisation par combinaison des différentes probabilités calculées.

L'invention concerne également un support non-transitoire lisible par ordinateur stockant un programme qui, lorsqu'il est exécuté par un microprocesseur ou un système informatique, conduit le système à exécuter tout procédé tel que défini ci-dessus.

Étant donné que la présente invention peut être mise en œuvre dans un logiciel, la présente invention peut être incorporée sous la forme d'un code lisible par ordinateur destiné à être fourni à un appareil programmable sur tout support approprié. Un support tangible peut comprendre un support de stockage tel qu'un disque dur, une bande magnétique ou un dispositif de mémoire à semi-conducteurs et autres. Un support transitoire peut comprendre un signal tel qu'un signal électrique, un signal électronique, un signal optique, un signal acoustique, un signal magnétique ou un signal électromagnétique, par exemple un signal micro-ondes ou RF.

**Brève description des figures**

D'autres particularités et avantages de l'invention apparaîtront encore dans la description ci-après, illustrée par les figures ci-jointes qui en illustrent des exemples de réalisation dépourvus de tout caractère limitatif. Sur les figures :

la Figure 1 illustre un système de suivi de l'utilisation par un patient d'un inhalateur-doseur sous pression, selon des modes de réalisation de l'invention ;
la Figure 2 illustre schématiquement des blocs ou unités fonctionnels d'un dispositif utilisateur pour une mise en œuvre de l'invention ;
la Figure 3 illustre l'interaction entre des doigts d'un patient et une inhalateur-doseur sous pression ;
la Figure 4 illustre le détermination d'une bonne ou mauvaise synchronisation à partir de trois probabilités déterminées selon des modes de réalisation de l'invention ;
la Figure 4a illustre le détermination d'une bonne ou mauvaise synchronisation à partir de trois probabilités déterminées selon d'autres modes de réalisation de l'invention ; et
la Figure 5 illustre, à l'aide d'un ordinogramme, des étapes générales pour la mise en œuvre de l'invention selon certains modes de réalisation.

**Description détaillée de modes de réalisation**

La bonne utilisation des thérapeutiques inhalées est essentielle dans le traitement de l'asthme et de la BPCO chez l'adulte et l'enfant. Celle-ci est généralement garantie par le respect de consignes prodiguées par le corps médical, tel un médecin.

Des aides à la prise médicamenteuse ont été développées pour éduquer et rendre les patients plus autonomes vis-à-vis des médecins.

Le document WO 2019/122315 divulgue par exemple un système mis en œuvre par ordinateur pour instruire une prise médicamenteuse d'un patient utilisant un dispositif pour inhaler un aérosol thérapeutique, puis commenter ou fournir un retour d'information sur cette prise médicamenteuse.

Comme indiqué dans ce document, un inhalateur est un dispositif d'inhalation capable d'émettre un aérosol thérapeutique permettant à un utilisateur ou à un patient d'inhaler l'aérosol. Un aérosol est une dispersion d'une phase solide, semi-solide ou liquide dans une phase gazeuse continue, comprenant donc par exemple les aérosols en poudre - connus sous le nom pharmaceutique de poudres pour inhalation - et les aérosols nébulisés. Les dispositifs d'inhalation pour l'administration d'aérosols en poudre sont communément décrits comme des inhalateurs de poudre. Les liquides en aérosol sont administrés au moyen de divers dispositifs d'inhalation, notamment des nébuliseurs, des inhalateurs-doseurs sous pression et des inhalateurs à brouillard doux.

Une difficulté existe pour le suivi de la prise médicamenteuse en cas d'utilisation des inhalateurs-doseurs sous pression, également connus sous l'appellation d'inhalateurs pMDI ou aérosols doseurs pressurisés. En effet, ceux-ci exigent du patient une attention particulière à la bonne synchronisation entre l'actionnement de l'inhalateur et sa propre inspiration, ce qui peut être difficile pour un patient commençant le traitement ou pour certaines franges de la population.

L'inhalateur-doseur sous pression comprend une cartouche de liquide en aérosol enfichée dans une tête (ou support de cartouche) portant un embout buccal. L'actionnement de l'inhalateur par simple appui relatif de la cartouche vers la tête, comprimant ainsi l'inhalateur, délivre une dose d'aérosol que le patient inhale, en sortie de l'embout buccal, par inspiration.

La présente invention améliore les techniques de détection d'une bonne ou mauvaise synchronisation en analysant, éventuellement en temps réel ou quasi-temps réel, des signaux vidéo et audio capturés pendant la prise médicamenteuse.

Des traitements sont réalisés sur des trames vidéos filmant le patient pour qualifier l'actionnement de l'inhalateur-doseur sous pression selon deux critères mais également sur un signal audio enregistrant, en même temps, le patient aux fins de détecter ou non une inhalation du patient. Une corrélation temporelle des résultats permet alors de qualifier la synchronisation entre l'actionnement de l'inhalateur-doseur sous pression et l'inspiration du patient, et ainsi remonter au patient un bon usage ou un mésusage de l'inhalateur.

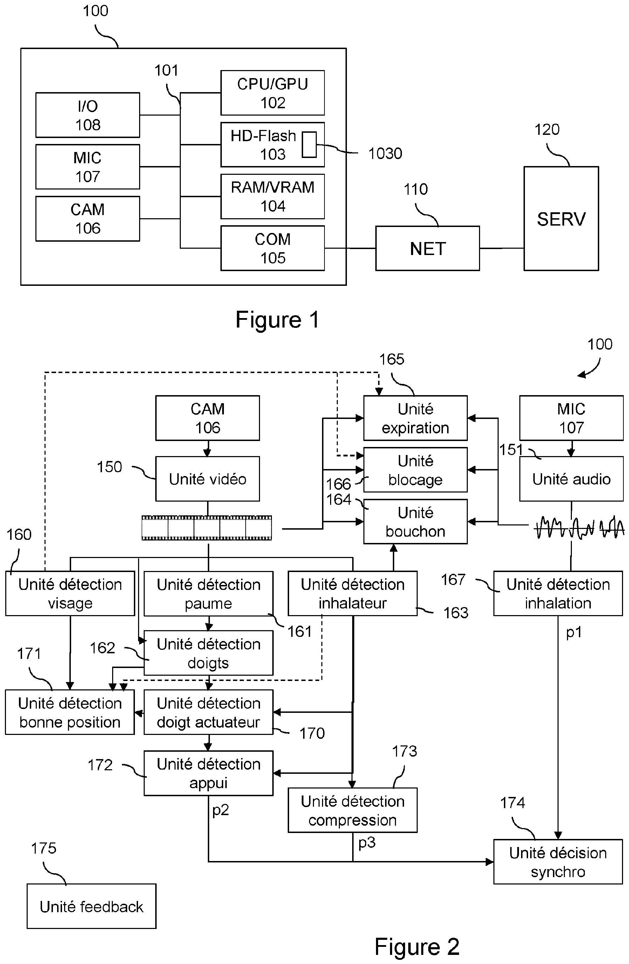

La Figure 1 illustre un système de suivi de l'utilisation par un patient d'un inhalateur-doseur sous pression, et donc son bon usage ou son mésusage.

Le système comprend un dispositif utilisateur 100 configuré pour mettre en œuvre certains modes de réalisation de l'invention. Le dispositif utilisateur 100 peut être un dispositif portatif tel qu'un téléphone intelligent (smartphone), une tablette numérique, un ordinateur portable, un assistant personnel, un dispositif de divertissement (e.g. console de jeu), ou être un dispositif fixe tel qu'un ordinateur de bureau ou une borne interactive, par exemple disposée au domicile ou dans un espace public tel une pharmacie ou un centre médical. Plus généralement, tout dispositif informatique aptes à la mise en œuvre des traitements évoqués ci-dessous peut être utilisé.

Le dispositif 100 comprend un bus de communication 101 auquel sont préférablement connectés :

- une ou plusieurs unités centrales de traitement 102, telles qu'un ou des processeurs CPU et/ou un ou des processeurs ou cartes graphiques GPU et/ou un ou des microprocesseurs ;
- une mémoire de stockage 103, de type ROM et/ou disque dur et/ou mémoire flash, pour le stockage de programmes informatiques 1030 destinés à mettre en œuvre l'invention mais également de toute donnée nécessaire à l'exécution des programmes ;
- une mémoire vive 104, de type RAM voire vidéo RAM (VRAM), pour le stockage du code exécutable des programmes informatiques ainsi que les registres adaptés pour enregistrer des variables et des paramètres nécessaires à leur exécution ;
- une interface de communication 105 connectée à un réseau externe 110 afin de communiquer avec un ou des serveurs distants 120 dans certains modes de réalisation de l'invention ;
- un dispositif de capture vidéo 106, typiquement une caméra intégrée ou rapportée, apte à capturer une séquence ou signal vidéo du patient utilisant l'inhalateur-doseur sous pression. Le dispositif de capture vidéo 106 peut être formé d'une seule caméra ou d'une matrice de caméras. Typiquement, un dispositif de capture vidéo 106 a une fréquence d'images (ou de trames) de 20, 25, 30, 50 voire plus images par seconde ;
- un dispositif de capture audio 107, typiquement un microphone intégré ou rapporté, apte à capturer une séquence ou signal audio du patient utilisant l'inhalateur-doseur sous pression. Le dispositif de capture audio 107 peut être formé d'un seul microphone ou d'une matrice de microphones ;
- une ou plusieurs entrées/sorties I/O 108 complémentaires permettant au patient d'interagir avec les programmes 1030 de l'invention en cours d'exécution. Typiquement, les entrées/sorties peuvent inclure un écran servant d'interface graphique avec le patient et/ou un clavier ou tout autre moyen de pointage permettant au patient de lancer par exemple l'exécution des programmes 1030 et/ou un haut-parleur. L'écran ou le haut-parleur peuvent servir de sortie pour fournir au patient un retour d'information sur la prise médicamenteuse telle qu'analysée par les programmes 1030 selon l'invention.

De préférence, le bus de communication assure la communication et l'interopérabilité entre les différents éléments inclus dans le dispositif informatique 100 ou connectés à celui-ci. La représentation du bus n'est pas limitative et, en particulier, l'unité centrale est utilisable pour communiquer des instructions à tout élément du dispositif informatique 100 directement ou au moyen d'un autre élément du dispositif informatique 100.

Le code exécutable stocké en mémoire 103 peut être reçu au moyen du réseau de communication 110, via l'interface 105, afin d'y être stocké avant exécution. En variante, le code exécutable 1030 n'est pas stocké en mémoire non volatile 103 mais peut être chargé en mémoire volatile 104 depuis un serveur distant via le réseau de communication 110 pour exécution directement. C'est le cas notamment des applications web (web apps).

L'unité centrale 102 est de préférence adaptée pour contrôler et diriger l'exécution des instructions ou des parties de code logiciel du ou des programmes 1030 selon l'invention. À la mise sous tension, le ou les programmes qui sont stockés en mémoire non volatile 103 ou sur le serveur distant sont transférés/chargés dans la mémoire vive 104, qui contient alors le code exécutable du ou des programmes, ainsi que des registres pour le stockage des variables et des paramètres nécessaires à la mise en œuvre de l'invention.

Dans un mode de réalisation, les traitements selon l'invention sont réalisés localement par le dispositif utilisateur 100, de préférence en temps réel ou quasi-temps réel. Dans ce cas, les programmes 1030 en mémoire mettent en œuvre l'ensemble des traitements décrits ci-après.

Dans une variante, une partie des traitements est déportée dans un ou plusieurs serveurs 120 éventuellement d'informatique en nuage (« cloud computing »), typiquement les traitements sur les signaux vidéo et audio. Dans ce cas, tout ou partie de ces signaux, éventuellement filtrés, sont transmis via l'interface de communication 105 et le réseau 110 jusqu'au serveur, lequel retourne en réponse certaines informations telles que les probabilités discutées ci-après ou la simple information représentative du degré de synchronisation ou encore le signal à remonter au patient. Les programmes 1030 mettent alors en œuvre une partie de l'invention, des programmes complémentaires prévus sur le ou les serveurs mettant en œuvre l'autre partie de l'invention.

Le réseau de communication 110 peut être tout réseau informatique filaire ou non-filaire ou un réseau de téléphonique mobile permettant une connexion à un réseau informatique tel que l'Internet.

La Figure 2 illustre schématiquement des blocs ou unités fonctionnels du dispositif 100 pour une mise en œuvre de l'invention. Comme indiqué plus haut, certaines de ces unités fonctionnelles peuvent être prévues dans le serveur 120 lorsqu'une partie des traitements y est déportée.

L'unité vidéo 150 adossée à la ou les caméras 106 enregistre le signal vidéo capturé dans une des mémoires du dispositif, typiquement en mémoire RAM 104 pour un traitement en temps réel ou quasi-temps réel. Cet enregistrement consiste notamment à enregistrer chaque trame vidéo du signal. A cette occasion, chaque trame est horodatée à l'aide d'une horloge interne (non représentée) du dispositif 100. L'horodatage permet une corrélation temporelle finale des informations obtenues par les traitements décrits ci-après.

Dans un mode de réalisation visant à réduire la charge de traitement, un sous-ensemble seulement des trames peut être enregistré et traité, typiquement 1 ou N-1 trames toutes les N trames (N entier, par exemple 2, 3, 4, 5 ou 10).

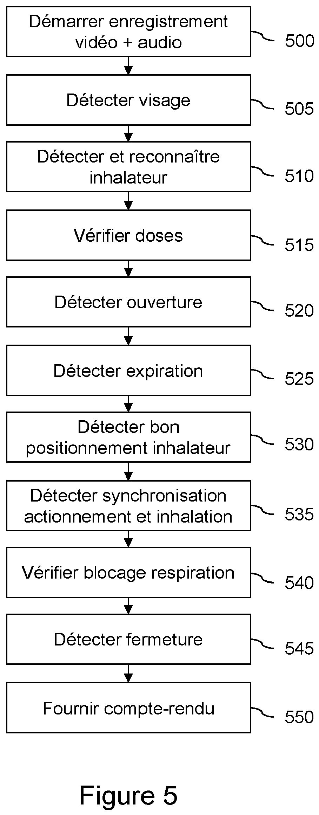

De façon symétrique, l'unité audio 151 adossée au ou aux microphones 107 enregistre les signaux audio capturés dans une des mémoires du dispositif, typiquement en mémoire RAM 104. L'unité audio 151 peut typiquement prétraiter le signal audio aux fins de créer des segments audio pour les traitements ultérieurs. La longueur (temporelle) des segments peut varier dynamiquement selon le traitement à appliquer, donc selon l'état d'avancement de l'algorithme décrit par la suite (Figure 5).

Par exemple, des segments de longueur 1 seconde peuvent être créés pour traitement par l'unité 164 de détection de l'ouverture ou fermeture du capuchon de l'inhalateur-doseur sous pression. En revanche, des segments plus longs, typiquement de longueur 2 à 10 s, de préférence 3 à 5 s, idéalement environ 3 s, sont créés et enregistrés en mémoire pour traitement par les unités de détection des expiration 165, inhalation 167 et blocage de la respiration 166.

De façon générale, des segments audio de longueur sensiblement égale à 3s peuvent être prévus pour l'ensemble de l'algorithme.

Des segments audio successifs peuvent se chevaucher. Ils sont par exemple générés avec un pas de génération compris entre 1/10s et 1s, par exemple 0,5s. Préférablement les segments audio sont alignés avec des trames vidéos, par exemple le milieu d'un segment audio correspond à une trame vidéo (dans une tolérance prédéfinie, par exemple 1/100 s pour une fréquence d'images de 25 im/s).

De façon similaire aux trames vidéo, chaque segment audio est horodaté, typiquement avec la même étiquette que la trame vidéo correspondant (ou la plus proche) au centre du segment audio. Bien entendu, d'autres correspondances entre trame vidéo, segment audio et horodatage peuvent être envisagées.

Chaque trame vidéo est fournie en entrée de l'unité de détection de visage 160, de l'unité de détection de paume 161, de l'unité de détection de doigts 162, de l'unité de détection de l'inhalateur 163 et de l'unité de détection de l'ouverture ou fermeture de l'inhalateur 164, optionnellement de l'unité de détection d'une expiration 165 et de l'unité de détection d'un blocage de la respiration 166.

Chaque segment audio est fourni en entrée de l'unité de détection de l'ouverture ou fermeture de l'inhalateur 164, de l'unité de détection d'une expiration 165, de l'unité de détection d'un blocage de la respiration 166 et de l'unité de détection d'une inhalation 167.

L'unité de détection de visage 160 peut être basée sur des techniques connues de reconnaissance de visage au sein d'images, typiquement des techniques de traitement de l'image. Selon un mode de réalisation, l'unité 160 met en œuvre un pipeline d'apprentissage automatique ou modèles d'apprentissage automatique ou machine (machine learning) supervisé. Un tel pipeline est entraîné à identifier des points de repère faciaux 3D.

De façon connue, un pipeline ou un modèle d'apprentissage automatique supervisé peut être de régression ou de classification. Des exemples de tels pipelines ou modèles incluent les forêts d'arbres décisionnels ou forêts aléatoires (« random forest »), les réseaux de neurones, par exemple convolutifs, les machines à vecteurs de support (Support Vector Machines - SVM).

Typiquement, des réseaux de neurones convolutifs peuvent être utilisés pour cette unité 160 (et les autres unités par la suite basées sur un pipeline ou modèle d'apprentissage automatique).

La publication « Real-time Facial Surface Geometry from Monocular Video on Mobile GPUs » (Yury Kartynnik et al) décrit typiquement un modèle de bout en bout basé sur un modèle de réseau neuronal pour déduire une représentation 3D approximative d'un visage humain, en 468 points de repères 3D, à partir d'une seule entrée de caméra (i.e. une seule trame). Il est en particulier bien adapté pour un traitement par des cartes graphiques de terminaux mobiles (donc aux ressources limitées). Les 468 points de repères 3D comportent notamment des points représentatifs de la bouche du visage.

L'unité de détection de visage 160 peut également être configurée pour effectuer un suivi (poursuite ou « tracking ») du visage dans des trames successives. Un tel suivi permet de résoudre certaines difficultés de détection dans une image suivante (occultation partielle du visage). Par exemple, la non détection soudaine d'un visage dans une trame vidéo peut être remplacée par une interpolation (e.g. linéaire) du visage entre une trame antérieure et une trame postérieure.

L'unité de détection de paume 161 peut également être basée sur des techniques connues de reconnaissance de main ou paume au sein d'images, typiquement des techniques de traitement de l'image. Selon un mode de réalisation, l'unité 161 met en œuvre un pipeline d'apprentissage automatique, par exemple à base de réseau de neurones convolutifs. Un tel pipeline est entraîné à identifier des points de repère 3D de la main.

La publication « MediaPipe Hands: On-device Real-time Hand Tracking » (Fan Zhang et al.) décrit une solution applicable. A nouveau, l'unité de détection de paume 161 peut être configurée pour effectuer un suivi (poursuite ou « tracking ») afin de corriger certaines difficultés de détection dans une trame donnée.

L'unité de détection de doigts 162 s'appuie sur la détection de la paume par l'unité 161 pour identifier et modéliser, par exemple en 3D, les points de repère 3D des doigts de la main. Des traitements classiques de l'image peuvent être mis en œuvre (recherche de modèles de main dans l'image autour de la paume localisée). Selon un mode de réalisation, l'unité 162 met en œuvre un pipeline d'apprentissage automatique, par exemple à base de réseau de neurones convolutifs. Un tel pipeline est entraîné à identifier des points de repère 3D des doigts.

L'unité 162 peut recevoir en entrée la trame vidéo rognée dans un voisinage de la paume identifiée par l'unité 161. Cette zone de voisinage ou région d'intérêt est connue sous l'appellation de « bounding box », et est dimensionnée pour englober l'entièreté de la main dont la paume a été identifié.

La publication « MediaPipe Hands: On-device Real-time Hand Tracking » ci-dessus décrit une solution applicable. A nouveau, l'unité de détection de doigts 162 peut être configurée pour effectuer un suivi (poursuite ou « tracking ») afin de corriger certaines difficultés de détection dans une trame donnée (par exemple un doigt caché).

Typiquement, les points de repère 3D des doigts de la main comprennent les articulations des phalanges (articulation à la base de chaque doigt, articulations entre phalanges) et le bout des doigts, ainsi qu'un lien entre chacun de ces points, identifiant ainsi la chaine de points formant chaque doigt et permettant leur suivi.

Les unités de détection de paume 161 et de détection de doigts 162, si elles sont représentées distinctes sur la figure, peuvent être mise en œuvre ensemble, par exemple à l'aide d'un unique pipeline d'apprentissage automatique à base de réseau de neurones convolutifs.

L'unité de détection de l'inhalateur 163 peut être basée sur des techniques connues de reconnaissance d'objets connus au sein d'images, typiquement des techniques de traitement de l'image. Selon un mode de réalisation, l'unité 163 met en œuvre un pipeline d'apprentissage automatique, par exemple à base de réseau de neurones convolutifs. Un tel pipeline est entraîné à identifier différents modèles d'inhalateurs. Il peut être créé à partir d'un pipeline pré-entraîné partiellement (pour la reconnaissance d'objets) et entraîne in fine à l'aide d'un jeu de données spécifique aux inhalateurs.

Préférablement, l'unité 163 localise l'inhalateur dans la trame vidéo traitée (une région d'intérêt ou « bounding box » autour de l'inhalateur peut être définie), identifie une famille ou un modèle d'inhalateur (selon que les données d'apprentissage aient été labellisées par famille ou type spécifique d'inhalateur) et optionnellement son orientation par rapport à un axe directeur (par exemple un axe longitudinal pour un inhalateur-doseur sous pression).

Un modèle de régression produit un score, indicateur ou une probabilité de confiance/plausibilité sur une échelle continue (sortie du modèle). En variante, un modèle de classification produit un score, indicateur ou une probabilité de confiance/plausibilité sur une échelle discrète (sortie du modèle correspondant à un type ou une famille d'inhalateur).

Plusieurs modèles peuvent être utilisés pour la détection d'objets, par exemple faster R-CNN, Mask R-CNN, CenterNet, EfficientDet, MobileNet-SSD, etc.

La publication « SSD: Single Shot MultiBox Detector » (Wei Liu et al.) par exemple décrit un modèle de réseau de neurones convolutifs qui permet à la fois la localisation et la reconnaissance d'objets dans des images. La localisation est notamment possible grâce à l'évaluation de plusieurs « bounding boxes » de tailles et ratios fixés à différentes échelles de l'image. Ces échelles sont obtenues par passage de l'image d'entrée au travers de couches convolutives successives. Le modèle prédit ainsi à la fois le décalage des « bounding boxes » avec l'objet recherché ainsi que le degré de confiance de la présence d'un objet.

L'unité de détection de l'inhalateur 163 peut être configurée pour effectuer un suivi (poursuite ou « tracking ») de l'inhalateur dans des trames successives, afin de corriger certaines difficultés de détection dans une trame donnée.

L'unité de détection de l'ouverture ou fermeture de l'inhalateur 164 permet, lorsque l'inhalateur est pourvu d'un capuchon ou d'un volet de fermeture, de détecter si ce dernier est en place (inhalateur fermé) ou bien retiré/ouvert.

Cette unité 164 peut opérer sur les seules trames vidéo, sur les seuls segments audio ou sur les deux.

Des techniques de traitement de l'image, basées sur des modèles d'inhalateur avec ou sans capuchon/volet de fermeture, peuvent être utilisées sur les trames vidéos, éventuellement sur la région d'intérêt entourant l'inhalateur tel qu'identifié par l'unité 163. Selon un mode de réalisation, l'unité 164 met en œuvre un modèle de réseau de neurones convolutifs entraîné à effectuer une classification entre un inhalateur ouvert et un inhalateur fermé, dans les trames vidéo.

Aussi, un basculement vers un état d'ouverture (respectivement fermeture) est détecté lorsque la classification passe de « inhalateur fermé » pour des trames antérieures à « inhalateur ouvert » pour des trames ultérieures. La première trame ultérieure peut indiquer un instant temporel de l'ouverture.

Des techniques de traitement du signal permettent d'identifier, dans les segments audio, un son caractéristique de l'ouverture ou de la fermeture de l'inhalateur, typiquement un « clic » spécifique à un type d'inhalateur ou à une famille d'inhalateurs. Des modèles de signal audio peuvent être prédéfinis et recherchés dans les segments audio. En variante, des marqueurs (typiquement des paramètres tels que des Coefficients Mel-Frequency Cepstral) typiques de ces sons caractéristiques sont recherchés dans les segments analysés. Selon un mode de réalisation, l'unité 164 met en œuvre un modèle réseau de neurones convolutifs entraîné à effectuer une classification entre un son d'ouverture et un son de fermeture d'inhalateur, dans les segments audio.

Le modèle de réseau de neurones convolutifs est par exemple entraîné avec des spectrogrammes. Un modèle d'apprentissage classique est par exemple entraîné sur des marqueurs/indicateurs caractéristiques du son (MFCC par exemple).

Une corrélation temporelle entre les segments audio détectant l'ouverture (respectivement fermeture) de l'inhalateur et les trames vidéo révélant un basculement vers un état d'ouverture (resp. fermeture) de l'inhalateur (c'est-à-dire un nombre défini de trames autour ou juste après le basculement) permet de confirmer ou renforcer le niveau de confiance de la détection vidéo de l'ouverture ou fermeture de l'inhalateur.

Les unités de détection d'une expiration 165, d'un blocage de la respiration 166 et d'une inspiration/inhalation 167 analysent les segments audio pour y détecter une expiration/un blocage/une inspiration ou inhalation par le patient.

Elles peuvent mettre en œuvre de simples modèles de sons de référence ou des marqueurs (typiquement des marqueurs/paramètres tels que des Coefficients Mel-Frequency Cepstral) typiques de ces sons de référence qui sont recherchés dans les segments analysés. Selon un mode de réalisation, toutes ou partie de ces unités mettent en œuvre un modèle d'apprentissage automatique, typiquement un réseau de neurones convolutifs, entraîné à détecter le son de référence. Les trois sons de référence, expiration, blocage et inspiration/inhalation, étant par nature différents, les trois unités peuvent être entraînées de façon dissociée, avec des jeux de données distincts.

Préférablement, chaque segment audio est filtré à l'aide d'un filtre de Butterworth passe-haut, dont la fréquence de coupure est choisie suffisamment basse (par exemple 400 Hz) pour retirer des composantes du spectre gênantes. Le segment audio filtré est alors converti en spectrogramme, par exemple en mel-spectrogramme. L'apprentissage des modèles (e.g. réseaux de neurones convolutifs) est alors effectué sur de tels spectrogrammes annotés (données d'apprentissage).

Un modèle de régression produit un score, indicateur ou une probabilité de confiance/plausibilité sur une échelle continue (sortie du modèle). En variante, un modèle de classification produit un score, indicateur ou une probabilité de confiance/plausibilité sur une échelle discrète (sortie du modèle) qui classifie les segments audio en des segments comportant ou non le son recherché. Il en résulte donc un niveau, score, indicateur de confiance ou une probabilité, dite d'expiration, de blocage ou d'inhalation, que le patient réalise, dans le segment audio, une expiration prolongée, un blocage de la respiration ou une inspiration combinée au flux aérosol.

La probabilité d'inhalation est notée p1 sur la Figure.

Dans une version simple, le modèle d'apprentissage automatique pour détecter un blocage de la respiration est le même que celui pour détecter une expiration, les sorties étant inversées : une absence d'expiration équivaut au blocage de la respiration, alors qu'une expiration équivaut à l'absence de blocage de la respiration. Cela simplifie la complexité algorithmique des unités 165 et 166.

Dans une version encore plus simple, un même et seul modèle non-binaire peut être entrainé à apprendre plusieurs classes : expiration (pour l'unité 165), inspiration (pour l'unité 167), l'absence d'expiration/inspiration (pour l'unité 166), voire l'ouverture (débouchage) et la fermeture (bouchage) de l'inhalateur (pour l'unité 164). Ainsi, une probabilité pour chaque événement est accessible via un seul modèle pour chaque segment audio traité.

L'unité de détection d'une expiration 165 peut en outre comprendre un traitement vidéo apte à détecter une bouche ouverte.

Il peut s'agir d'un traitement de l'image. Par exemple, l'unité 165 reçoit en entrée les points de repère 3D de l'unité de détection de visage 160 pour la trame vidéo courante, détecte l'ouverture de la bouche lorsque les points 3D représentatifs des bords supérieur et inférieur de la bouche sont suffisamment éloignés.

En variante, un modèle d'apprentissage automatique, typiquement un réseau de neurones convolutifs entraîné, est mis en œuvre.

Une corrélation temporelle entre des trames vidéo successives révélant une bouche ouverte pendant une durée minimale (notamment entre 1 et 5s, par exemple 3s environ) et les segments audio détectant un son de référence d'expiration permet de confirmer ou renforcer le niveau/score/indicateur de confiance de la détection audio de l'expiration.

De même, l'unité de détection d'un blocage de la respiration 166 peut en outre comprendre un traitement vidéo apte à détecter une bouche fermée.

Il peut s'agir d'un traitement de l'image. Par exemple, l'unité 166 reçoit en entrée les points de repère 3D de l'unité de détection de visage 160 pour la trame vidéo courante, détecte une bouche fermée lorsque les points 3D représentatifs des bords supérieur et inférieur de la bouche sont suffisamment proches.

En variante, un modèle d'apprentissage automatique, typiquement un réseau de neurones convolutifs entraîné, est mis en œuvre.

Une corrélation temporelle entre des trames vidéo successives révélant une bouche fermée pendant une durée minimale (notamment entre 2 et 6s, par exemple 4 ou 5s) et les segments audio détectant un son de référence de blocage de respiration permet de confirmer ou renforcer le niveau/score/indicateur de confiance de la détection audio du blocage.

Le dispositif utilisateur 100 comporte en outre l'unité de détection du doigt actuateur 170, l'unité de détection d'une bonne position de l'inhalateur 171, l'unité de détection d'un appui 172, l'unité de détection de compression 173, l'unité de décision de synchronisation 174 et l'unité feedback 175.

L'unité de détection du doigt actuateur 170 reçoit en entrée les points de repère 3D des doigts (de l'unité 162) et les informations de localisation de l'inhalateur dans l'image (de l'unité 163).

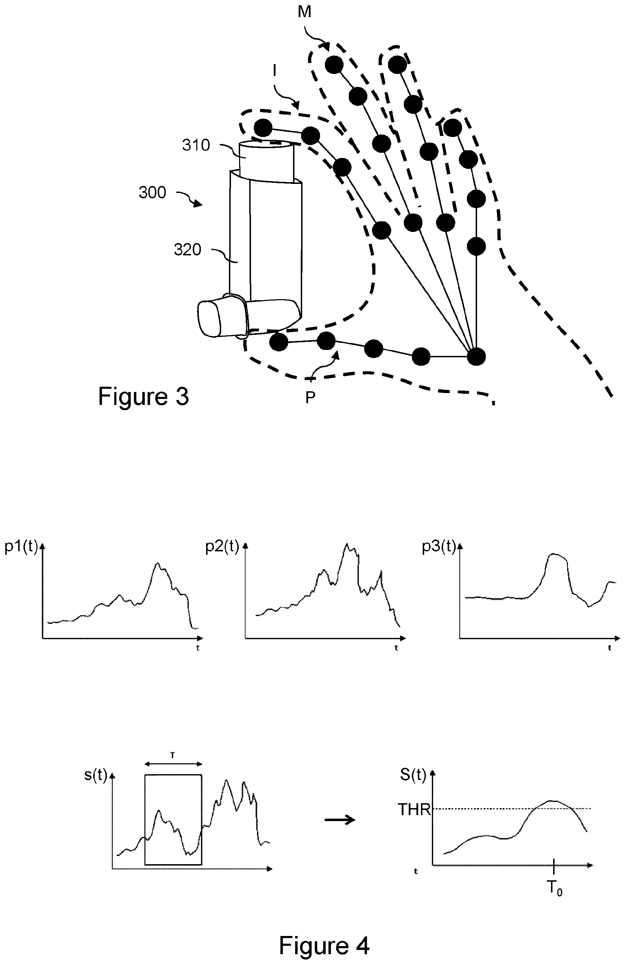

On s'intéresse ici aux inhalateurs-doseurs sous pression qui sont utilisés en position verticale inversée (ouverture vers le bas) comme montré en Figure 3.

La détection du ou des doigts actuateurs, c'est-à-dire ceux positionnés pour actionner l'inhalateur (en pratique presser sur la cartouche 310 relativement à la tête 320), par l'unité 170 peut être réalisée comme suit.

Les points de repère 3D de doigts présents dans la région d'intérêt autour de l'inhalateur (obtenue de l'unité 163) sont pris en compte et permettent une classification de la tenue de l'inhalateur en position verticale inversée (c'est-à-dire comment l'inhalateur est tenu par le patient).

Cette classification peut être réalisée par un simple algorithme relevant de considérations géométriques ou à l'aide d'un modèle d'apprentissage automatique, typiquement un réseau de neurones convolutifs.

Dans un exemple algorithmique, l'unité 170 détermine que le bout du pouce se trouve ou non sous la tête 320 et, dans l'affirmative, que l'extrémité de l'index est placée sur le fond de la cartouche 310. C'est le cas si le point de repère 3D de l'extrémité du pouce est détecté comme sensiblement situé dans le voisinage et en dessous de la tête inversée 320 alors que l'extrémité de l'index est détectée comme sensiblement située dans le voisinage et au-dessus de la cartouche inversée 310. Cette tenue correspond à une première classe C1.

D'autres classes Ci, prédéfinies en un nombre déterminé, peuvent être détectées, par exemple à titre illustratif et non exhaustif :

C2 : bout du pouce sous la tête 320 et extrémité du majeur sur fond de cartouche 310,

C3 : bout du pouce sous la tête 320 et extrémités de l'index et du majeur sur fond de cartouche 310,

C4 : extrémité de l'index sur fond de cartouche 310, les autres doigts entourant la tête,

C5 : extrémité du majeur sur fond de cartouche 310, les autres doigts entourant la tête,

C6 : inhalateur tenu à deux mains, extrémités de l'index et du majeur droits sur fond de cartouche 310, ...

A chaque classe est associé un doigt actuateur, typiquement le ou les doigts posés sur le fond de la cartouche 310. Cette information est stockée en mémoire. L'unité 170 réalisant la classification du mode de tenue de l'inhalateur est ainsi en mesure de retourner, en sortie, le ou les doigts actuateurs.

Par exemple, pour la classe C1, le doigt actuateur est l'index « I ». Pour la classe C2, il s'agit du majeur « M ». Pour la classe C3, il y a deux doigts actuateurs : l'index et le majeur.

L'unité de détection d'une bonne position de l'inhalateur 171 réalise un traitement des informations obtenues par les unités 160 (position du visage et de la bouche), 162 (position des doigts), 163 (position et orientation de l'inhalateur) et 170 (doigt actuateur).

La détection du bon ou mauvais positionnement de l'inhalateur-doseur sous pression peut simplement consister à classer (bon ou mauvais positionnement) une trame vidéo en tenant compte également la classe Ci de tenue de l'inhalateurs.

Cette classification peut être réalisée par un simple algorithme relevant de considérations géométriques ou à l'aide d'un modèle d'apprentissage automatique, typiquement un réseau de neurones convolutifs.

Dans un exemple algorithmique, pour les classes C1-C3, on vérifie que la main est placée à la verticale le pouce en bas, c'est-à-dire le point de repère 3D du bout du pouce « P » est situé plus bas que celui du ou des doigts actuateurs (index « I » et/ou majeur « M »), et la distance entre le point de repère 3D du bout du/des doigts actuateurs et le point de repère 3D du bout du pouce « P » est supérieure à une valeur seuil (fonction de la dimension de l'inhalateur déterminée par exemple par l'unité 163 identifiant le type ou la famille d'inhalateur dans les trames vidéo).

En outre, le point de repère 3D du bout du pouce « P » ne doit pas être situé plus bas qu'un certain seuil à compter du point de repère 3D des points milieux de la bouche tels que fournis par l'unité 160 et/ou le bas de la tête 320 de l'inhalateur en position verticale inversée doit être placé près de la bouche, i.e. à un certain seuil du point milieu de la bouche. Cette condition vérifie que l'embout buccal de la tête 320 est à hauteur de la bouche.

Enfin, l'unité 171 vérifie que les lèvres sont bien refermées autour de l'inhalateur, i.e que la distance entre le point milieu inférieur et le point milieu supérieur de la bouche (tels que fournis par l'unité 160) est inférieure à un certain seuil.

L'unité 171 peut vérifier ces conditions sur les trames vidéo successives et n'émettre une validation de bon positionnement que lorsqu'elles sont validement vérifiées sur un certain nombre de trames vidéo consécutives.

Le respect plus ou moins fort de ces seuils permet de graduer un niveau, score, indicateur ou probabilité que les conditions soient vérifiées, c'est-à-dire que l'inhalateur soit bien positionné.

De même, l'utilisation d'un modèle d'apprentissage automatique permet soit de classer binairement les trames vidéo en « position correcte » ou « position incorrecte », soit de fournir un niveau, score, indicateur ou probabilité plus nuancé.

L'unité de détection d'un appui 172 vérifie si le ou les doigts actuateurs sont en phase d'appui sur la cartouche 310 de l'inhalateur-doseur sous pression. L'unité 172 reçoit en entrée les points de repère 3D du ou des doigts actuateurs (des unités 162 et 170).

Lorsque l'unité 172 est activée pour une phase de détection d'un appui, elle enregistre une position de référence des points de repère 3D du ou des doigts actuateurs, par exemple la première position reçue. Il s'agit typiquement d'une position sans appui, qui permet, comme décrit par la suite, d'évaluer l'amplitude de l'appui dans chaque trame ultérieure.

L'unité 172 détermine ensuite le mouvement de l'extrémité du ou des doigts actuateurs par rapport à cette position de référence. Il s'agit typiquement, pour un appui, de déterminer un mouvement relatif de descente du bout du doigt actuateur par rapport à une base du doigt (articulation de la première phalange avec la main), en comparaison de la position de référence.

Le mouvement relatif de descente (distance longitudinale de descente, typiquement verticale) peut être comparé à une course maximale de compression de la cartouche de l'inhalateur.

Une course réelle maximale peut être obtenue au travers de l'identification de l'inhalateur-doseur sous pression (chaque inhalateur ayant une course réelle connue) puis être convertie en course maximale dans la trame vidéo en cours de traitement. Aussi, le ratio entre la distance longitudinale mesurée de descente de l'extrémité du doigt actuateur et la course maximale de trame est représentatif d'un niveau, score, indicateur de confiance ou d'une probabilité (dite d'appui) que le patient dans la trame vidéo est en phase d'appui (c'est-à-dire enfonce) sur un élément déclencheur de l'inhalateur-doseur sous pression. Cette probabilité d'appui, notée p2 sur la Figure 2, est émise en sortie de l'unité 172.

Cet exemple ne tient compte que du mouvement de l'extrémité du doigt actuateur. Des modèles plus complexes vérifiant également le mouvement des phalanges du même doigt peuvent être pris en compte afin notamment de détecter (en terme de probabilité) un mouvement particulier de courbure descendante de l'extrémité du doigt.

En variante, un ensemble de profils correspondant à plusieurs positions des doigts selon l'intensité de l'appui peut être stocké en mémoire et comparé à la trame courant pour déterminer un profil le plus proche, et par voie de conséquence, une amplitude d'appui (ainsi une probabilité d'appui).

En variante d'une approche algorithmique, un modèle d'apprentissage automatique (entrainé) peut être utilisé.

L'unité de détection de compression 173 renseigne de l'état de compression de l'inhalateur-doseur sous pression. En effet, l'actionnement de l'inhalateur est réalisé par simple appui relatif de la cartouche 310 dans la tête 320. L'analyse des trames vidéo permet de générer un niveau, score, indicateur de confiance ou une probabilité (dite de compression) que l'inhalateur-doseur sous pression dans la trame vidéo est dans un état comprimé. Cette probabilité de compression est notée p3 sur la Figure 2.

L'unité 173 reçoit en entrée la détection de l'inhalateur (région d'intérêt identifiée et type ou famille d'inhalateur). Le type ou la famille d'inhalateur permet de récupérer la dimension (typiquement longueur) réelle de l'inhalateur dans un état non comprimé et sa dimension réelle dans un état comprimé. Ces dimensions peuvent être représentatives de la longueur totale de l'inhalateur ou en variante de la longueur de la partie visible de la cartouche. Ces dimensions sont converties en dimensions vidéo dans la trame vidéo en cours de traitement (par exemple en multipliant chaque longueur réelle par le ratio entre la dimension de la tête dans la trame et la dimension réelle de la tête 320).

La longueur mesurée sur la trame vidéo courante est alors comparée aux longueurs de référence correspondant aux états comprimé et non comprimé pour attribuer (par exemple de façon linéaire) une probabilité comprise entre 0 (état non comprimé) et 1 (état comprimé).

Dans une variante, l'unité 173 met en œuvre un modèle d'apprentissage automatique, typiquement un réseau de neurones entraîné, prenant en entrées la région d'intérêt autour de l'inhalateur et classifiant ce dernier en deux catégories : inhalateur comprimé et inhalateur non comprimé. L'unité 173 peut notamment être mise en œuvre conjointement avec l'unité 163, c'est-à-dire en utilisant le même réseau de neurones apte à détecter un inhalateur dans une trame vidéo, à catégoriser cet inhalateur, à délimiter une région d'intérêt autour de l'inhalateur et à qualifier l'état (une probabilité entre 0 et 1 représentant les états comprimé et non comprimé) de l'inhalateur pour le cas où l'inhalateur serait un inhalateur-doseur sous pression.

Dans ce mode de réalisation, l'unité 173 prend en entrée l'imagette en sortie de l'unité 163, contenant l'inhalateur, et renvoie sa probabilité d'être en état compressé. Pour cela, un réseau de neurones convolutifs pour la classification est entrainé sur une base d'images d'inhalateurs compressé et non compressés. Le réseau est choisi d'une architecture simple comme LeNet-5 (Y. LeCun, L. Bottou, Y. Bengio, and P. Haffner. Gradient-based learning applied to document récognition. Proceedings of the IEEE, november 1998), et est entraîné par descente de gradient par batches, avec réduction du taux d'apprentissage pour assurer la bonne convergence du modèle.

L'unité 174 est une unité de décision de la bonne ou non synchronisation entre l'actionnement de l'inhalateur-doseur sous pression et une inspiration du patient. Elle utilise les probabilités d'appui p2, de compression p3 et d'inhalation p1 correspondant à des mêmes instants temporels, comme décrit par la suite.

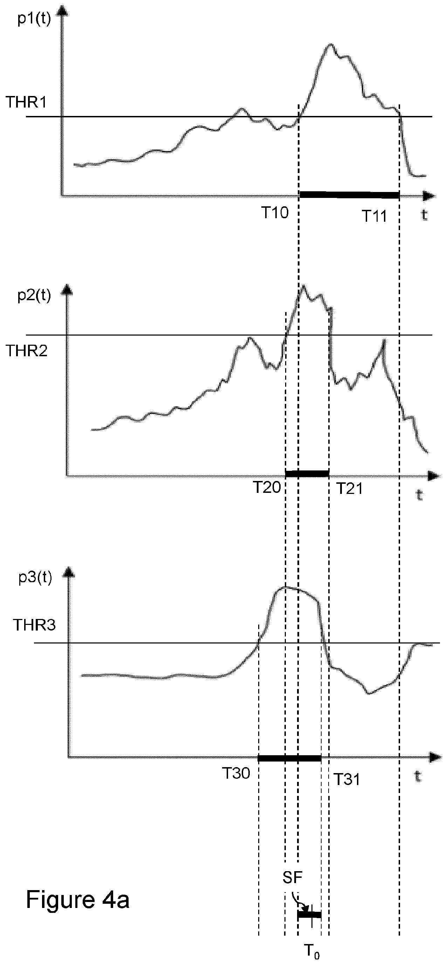

Dans un mode de réalisation, ces probabilités sont combinées, par exemple linéairement, pour chacun d'une pluralité d'instants temporels. Un exemple de probabilités p1, p2, p3 dans le temps est illustré en Figure 4. L'échantillonnage de la probabilité p1 (pas temporel entre chaque segment) peut être différent de celui des probabilités p2 et p3 (fréquence des trames vidéo traitées). Le cas échéant, une interpolation est réalisée pour obtenir une valeur pour les trois probabilités à chaque instant temporel considéré.

Les instants considérés peuvent correspondre à la plus petite période d'échantillonnage entre les trois probabilités, donc préférablement à chaque trame traitée. Bien entendu, pour alléger le traitement, un sous-ensemble de ces instants peut être considéré.

A titre d'exemple, la probabilité combinée à l'instant t est notée s(t) :

$$s(t) = a.p1(t) + b.p2(t) + c.p3(t)$$

Elle peut être optionnellement moyennée sur une fenêtre glissante de largeur Tmoy donnant un score global ou un degré de synchronisation S(t), comme illustré sur la Figure 4. En variante, un moyennage sur fenêtre glissante peut être réalisé sur chacune des probabilités p1, p2 et p3 avant combinaison en s(t). Dans ce cas, une même taille Tmoy de fenêtre peut être utilisée ou en variante différentes tailles Tmoy1, Tmoy2 et Tmoy3 de fenêtre peuvent être utilisées respectivement pour les probabilités p1, p2 et p3.

L'unité 174 peut alors comparer le score global à une valeur seuil THR à partir de laquelle une synchronisation correcte est détectée.

Les paramètres a, b, c, Tmoy (voire Tmoy1, Tmoy2 et Tmoy3) et THR peuvent être appris par validation croisée sur des vidéos et signaux sonores de bons et mauvais usages.

Dans l'exemple de la Figure 4, le score S(t) de synchronisation montre que le patient a réalisé une bonne synchronisation entre l'actionnement de l'inhalateur et son inspiration, aux environs de l'instant To.

Si le score S(t) ne dépasse pas la valeur seuil THR dans la fenêtre d'analyse de l'étape 535, il peut être déterminé que la synchronisation n'a pas été bonne.

Dans un autre mode de réalisation à la combinaison des probabilités en un score global, il est déterminé pour chaque probabilité p1, p2, p3 s'il existe une fenêtre temporelle de haute probabilité, respectivement d'inhalation, d'appui et de compression. La haute probabilité peut simplement consister en une valeur seuil pour chaque probabilité considérée. Si plusieurs fenêtres sont identifiées pour une probabilité données, la plus large peut être conservée.

En référence à la Figure 4a par exemple, un seuil THR1 permet de déterminer une fenêtre temporelle (T10, T11) où la probabilité d'inhalation est forte ; un seuil THR2 permet de déterminer une fenêtre temporelle (T20, T21) où la probabilité d'appui est forte ; et un seuil THR3 permet de déterminer une fenêtre temporelle (T30, T31) où la probabilité de compression est forte.

Le recouvrement temporel des fenêtres est alors analysé pour déterminer un degré de synchronisation entre l'actionnement de l'inhalateur et l'inspiration du patient. Il s'agit ainsi de corréler temporellement les probabilités obtenues précédemment.

Par exemple, on détermine la sous-fenêtre SF commune aux trois fenêtre temporelles.

Dans une variante, on détermine la plus grande sous-fenêtre commune entre la fenêtre temporelle (T10, T11) et l'une des deux autres fenêtres temporelles. On corrèle ainsi la probabilité (d'inhalation) issue de l'analyse audio avec une probabilité issue de l'analyse vidéo. Cette variante permet de surmonter d'éventuelles difficultés à analyser la compression de l'inhalateur (par exemple s'il est fortement occulté par les mains du patient) ou l'appui du patient.

La présence d'une sous-fenêtre de chevauchement permet par exemple d'indiquer une bonne synchronisation.

Dans un mode de réalisation, l'unité 174 vérifie que la sous-fenêtre a une durée minimale (notamment entre 1s et 3s) avant d'indiquer une bonne synchronisation. Cela réduit les risques d'une détection inopinée.

Dans l'exemple de la Figure 4a, le chevauchement des fenêtres temporelles montre que le patient a bien synchronisé l'actionnement de l'inhalateur et son inspiration, aux environs de l'instant To.

Dans un mode de réalisation, les probabilités p1, p2, p3 sont moyennées sur une fenêtre temporelle prédéfinie, avant détermination des fenêtres temporelles (T10, T11), (T20, T21) et (T30, T31).

Ces approches corrélant les probabilités p1, p2, p3 sont avantageusement robustes à l'absence de certaines probabilités (mauvaise détection dans des trames par exemple). Certaines probabilités manquantes peuvent être interpolées à partir de probabilités existantes à des instants suffisamment proches. De même, p2 ou p3 peuvent être corrélées à p1 sans l'autre.

Le dispositif utilisateur 100 comporte enfin une unité feedback 175 faisant un retour au patient sur l'analyse de la prise médicamenteuse. Ce retour comporte notamment un signal de bon usage ou de mésusage de l'inhalateur-doseur sous pression au patient tel que déterminé par l'unité 174. D'autres informations peuvent être remontées également, comme par exemple des erreurs détectées (mauvais positionnement, inhalateur non ouvert, mauvaise expiration/blocage de la respiration, etc.).

Chaque retour peut être restitué en temps réel ou quasi-réel, c'est-à-dire lorsqu'il est généré par une unité fonctionnelle active durant une phase particulière du procédé décrit par la suite. En variante, les retours peuvent être restitués à la fin du procédé, auquel cas ils sont mémorisés en mémoire au fil de leur création (pendant les différentes phases du procédé). Les deux alternatives peuvent être combinées : présentation des retours à leur génération et en fin de procédé.

Chaque retour peut être restitué visuellement (écran du dispositif 100) ou oralement (haut-parleur) ou les deux.

Comme indiqué ci-dessus, certaines unités peuvent être mises en œuvre à l'aide de modèles d'apprentissage automatique supervisés, typiquement des réseaux de neurones entraînés. L'apprentissage de tels modèles à partir de données d'apprentissage est bien connu de l'homme de l'art et donc non détaillé ici. Les probabilités générées par les unités de traitement sont préférentiellement comprises entre 0 et 1, afin de simplifier leur manipulation, combinaison et comparaison.

La Figure 5 illustre, à l'aide d'un logigramme, des étapes générales d'un procédé de suivi de l'usage ou utilisation par un patient d'un inhalateur-doseur sous pression. Ces étapes utilisent les unités de traitement décrites ci-dessus.

Ce procédé peut par exemple être mis en œuvre au moyen d'un programme informatique 1030 (application) exécuté par le dispositif 100. A titre d'exemple, le patient utilise une tablette numérique et lance l'application selon l'invention. Cette application peut proposer une procédure d'accompagnement pas à pas (avec affichage de chacune des actions à mener telles que décrites ci-après) ou laisser le patient effectuer sa prise médicamenteuse seule, sans indication.

Le procédé démarre au lancement de l'exécution du programme. Le procédé permet au programme de passer successivement dans plusieurs états d'exécution, chaque état correspondant à une étape. Chaque état peut n'être activé que si l'état précédent est validé (soit par détection positive soit par expiration d'un temps prédéfini ou temporisation). Dans chaque état, certaines unités sont actives (pour les besoins de l'étape correspondante), d'autres non, limitant ainsi l'utilisation des ressources de traitement.

Une indication de l'état courant peut être fournie au patient, par exemple l'état (c'est à dire la phase ou opération en cours du procédé) est affiché sur l'écran. De même, les retours sur la bonne réalisation d'une phase donnée ou sur l'existence d'une erreur peuvent être fournis au patient en temps réel, par exemple affichés sur l'écran.

A l'étape 500, les enregistrements vidéo et audio par les unités 150 et 151 via la caméra 105 et le microphone 107 sont démarrés. Chaque trame acquise est stockée en mémoire, de même que le signal audio possiblement converti en plusieurs segments audio.

A l'étape 505, le procédé entre dans l'état « détection de visage ». L'unité 160 est activée permettant de détecter un visage sur les trames vidéo. Dès qu'un visage est détecté sur plusieurs trames vidéo successives (par exemple un nombre prédéfini), l'étape est validée. Sinon l'étape perdure jusqu'à expiration d'une temporisation.

Le procédé passe à l'état « détection de l'inhalateur » à l'étape 510. L'unité 163 est activée permettant de détecter un inhalateur, de le localiser et de déterminer son type ou sa famille. Cela permet de récupérer des informations utiles pour les étapes suivantes (course maximale, classes de tenue de l'inhalateur, ...).

Si l'inhalateur n'est pas de type inhalateur-doseur avec pression, le procédé peut se poursuivre comme dans les techniques connues.

Si l'inhalateur est de type inhalateur-doseur avec pression, son modèle ou sa famille est reconnu et mémorisé en mémoire.

Le procédé passe à l'état « détection des doses restantes » à l'étape 515 si le modèle reconnu d'inhalateur présente un compteur de doses, sinon (modèle non reconnu ou pas de compteur) il passe directement à l'étape 520.

A l'étape 515, l'unité 163 toujours activée réalise un suivi de l'inhalateur au travers des trames vidéo successives, détermine une sous-zone de l'inhalateur correspondant à l'indication des doses restantes (compteur ou dosimètre). Une fois cette sous-zone localisée, une analyse OCR (reconnaissance de caractères) est effectuée afin de déterminer s'il reste un nombre suffisant de doses (par exemple la valeur indiquée doit être différente de 0).

Dans la négative, le procédé peut s'arrêter avec un message d'erreur ou continuer en mémorisant cette erreur pour un affichage lors du compte-rendu final.

Dans l'affirmative, le procédé passe à l'état « détection de l'ouverture » à l'étape 520. Cette étape met en œuvre l'unité 164 qui est activée pour l'occasion. A nouveau un indicateur peut être affiché au patient tant que l'unité 164 ne détecte pas que l'inhalateur est ouvert.

Lorsque l'ouverture a été détectée ou après une temporisation, le procédé passe à l'état « détection d'une expiration profonde » à l'étape 525. L'unité 164 est désactivée. Cette étape 525 met en œuvre l'unité 165 qui est activée pour l'occasion. L'unité 165 effectue par exemple une corrélation temporelle entre la détection sonore d'une expiration profonde dans le signal audio et la détection d'une bouche ouverte dans le signal vidéo (par l'unité 160).

La probabilité (ou le score de confiance) d'expiration est mémorisée pour être indiquée au patient dans le compte-rendu final, notamment sur une échelle de 1 à 10.

Lorsqu'une expiration a été détectée ou après une temporisation (par exemple la phase d'expiration se tient en 5s environ), le procédé passe à l'état « détection du bon positionnement de l'inhalateur » à l'étape 530. L'unité 165 est désactivée. Cette étape 530 met en œuvre l'unité 171 décrite plus haut qui est activée pour l'occasion. Elle nécessite l'activation des unités 161, 162 et 170, l'unité 160 étant toujours activée. Aussi, ces premières unités ne commencent à traiter les trames vidéo qu'à partir de cette étape.

Un indicateur peut être affiché au patient lui indiquant tant que l'inhalateur est mal positionné, notamment dans le mauvais sens ou mal positionné relativement à la bouche du patient.

Cet indicateur peut disparaître lorsqu'un bon positionnement est détecté pour un nombre de trames vidéo consécutives. Le procédé passe alors à l'état « détection de la synchronisation d'inhalation » à l'étape 535.

Le procédé peut également passer dans cet état après expiration d'une temporisation quand bien même le bon positionnement n'ait pas été correctement validé (ce qui sera par exemple remonté au patient lors de l'étape finale 550).

Les étapes jusqu'ici permettent ainsi de déterminer le bon moment où procéder à la détection d'une bonne ou mauvaise synchronisation de l'actionnement de l'inhalateur et de l'inspiration/inhalation du patient. Cette étape de détection 535 est ainsi déclenchée par la détection d'un bon positionnement de l'inhalateur-doseur sous pression par rapport au patient dans des trames vidéos antérieures.

La phase d'inhalation par le patient dure en général moins de 5s, par exemple 3s, aussi une temporisation (de 5s) de l'étape peut être mise en place.

L'état « détection de la synchronisation d'inhalation » active les unités 167, 172 et 173 pour traiter les trames vidéo et les segments audio qui arrivent désormais, ainsi que l'unité 174.

L'unité 167 fournit les probabilités d'inhalation p1(t) tant que l'étape se poursuit.

L'unité 172 fournit les probabilités d'appui p2(t). L'unité 173 fournit les probabilités de compression p3(t).

L'unité 174 traite en temps réel ou au terme de la temporisation de l'étape l'ensemble des probabilités p1(t), p2(t) et p3(t) afin de déterminer le degré de synchronisation entre l'actionnement de l'inhalateur-doseur sous pression et une inspiration du patient comme décrit plus haut. Cette information est mémorisée en mémoire et/ou affichée au patient, via l'unité feedback 175.

Dans un mode de réalisation, l'étape 535 peut inclure une vérification permanente du bon positionnement comme réalisée à l'étape 530. Cela permet d'avertir le patient ou de mémoriser une erreur pour le cas où le patient modifie, de façon dommageable, le positionnement de son inhalateur.

A la fin de la temporisation ou en cas de détection d'un degré satisfaisant de synchronisation, le procédé passe à l'état suivant « détection de la retenue du souffle » à l'étape 540. C'est la fin de l'opération de détection d'une bonne ou mauvaise synchronisation.

Les unités 161, 162, 167, 170, 171, 172, 173 peuvent être désactivées, l'unité 160 étant maintenue active poursuivre l'état d'ouverture de la bouche, ainsi que l'unité 163. L'unité 166 est alors activée, traitant les segments audio arrivant et/ou les nouvelles trames vidéo, pour déterminer si le patient retient ou non son souffle pendant une durée suffisante. L'étape 540 dure quelques secondes (par exemple 5s) au terme de laquelle les unités 160 et 166 sont désactivées.

Le procédé passe alors à l'état « détection de la fermeture de l'inhalateur » à l'étape 545. Cette étape utilise l'unité 164 qui est à nouveau activée pour détecter la fermeture de l'inhalateur.

Une temporisation est prévue, notamment parce que le patient peut écarter l'inhalateur du champ de la caméra, empêchant toute détection de la fermeture.

Si une fermeture est détectée ou la temporisation expire, le procédé passe à l'étape suivante 550 dans l'état « compte-rendu ».

Dans un mode de réalisation, les étapes 540 et 545 sont réalisées en parallèle. En effet, il se peut que le patient referme l'inhalateur en même temps qu'il bloque sa respiration. Les unités 160, 163, 164 et 166 sont alors actives en même temps.

A l'étape 550, les unités encore actives, 163, 164, sont désactivées. L'unité feedback 175 est activée si besoin est, laquelle récupère en mémoire l'ensemble des messages/erreurs/indications mémorisées par les diverses unités activées pendant le procédé.

Les messages, incluant celui précisant le degré de synchronisation entre l'actionnement de l'inhalateur-doseur sous pression et une inspiration du patient, sont restitués au patient, par exemple par simple affichage sur l'écran du programme exécuté. Le compte-rendu peut notamment détailler le résultat de chaque étape, avec un niveau de réussite associé.

Bien que la description ci-dessus du procédé de la Figure 5 active et désactive les unités à la demande selon l'avancement du procédé, il peut être prévu que tout ou partie des unités soient actives au lancement du programme. Typiquement, l'unité feedback 175 peut être activée d'emblée afin de permettre une remontée d'information au patient en toute phase du procédé. Par ailleurs, les unités 160, 161, 162 et 163 peuvent également être activées d'emblée. Optionnellement, l'unité 170 l'est également. Subsidiairement, les unités 164, 165, 166 et 167 le sont également.

Les exemples qui précèdent ne sont que des modes de réalisation de l'invention qui ne s'y limite pas.

**Revendications**

1. Procédé mis en œuvre par ordinateur de suivi de l'usage, par un patient, d'un inhalateur-doseur sous pression (300), comprenant les étapes suivantes :

   obtenir un signal vidéo et un signal audio d'un patient utilisant un inhalateur-doseur sous pression,
   calculer, pour chacune d'une pluralité de trames vidéo du signal vidéo, au moins l'une parmi une probabilité, dite d'appui, (p2) qu'un doigt actuateur du patient dans la trame vidéo soit en phase d'appui sur un élément déclencheur (310) de l'inhalateur-doseur sous pression et une probabilité, dite de compression, (p3) que l'inhalateur-doseur sous pression dans la trame vidéo soit dans un état comprimé,
   calculer, pour chacun d'une pluralité de segments audio du signal audio, une probabilité, dite d'inhalation, (p1) que le patient réalise, dans le segment audio, une inspiration combinée au flux aérosol,
   déterminer (535) un degré de synchronisation entre l'actionnement de l'inhalateur-doseur sous pression et une inspiration du patient à partir des probabilités d'appui, de compression et d'inhalation correspondant à des mêmes instants temporels, et
   émettre (550) en conséquence un signal de bon usage ou de mésusage de l'inhalateur-doseur sous pression au patient.

2. Procédé selon la revendication 1, dans lequel déterminer un degré de synchronisation comprend déterminer, pour chaque type de probabilité, une fenêtre temporelle de haute probabilité, et le degré de synchronisation est fonction d'un recouvrement temporel des fenêtres temporelles ainsi déterminées pour les probabilités.

3. Procédé selon la revendication 1, dans lequel déterminer un degré de synchronisation comprend :

   combiner, pour chacun d'une pluralité d'instants temporels, les probabilités d'appui, de compression et d'inhalation correspondant audit instant temporel en une probabilité combinée, et
   déterminer, à partir des probabilités combinées, un degré de synchronisation entre l'actionnement de l'inhalateur-doseur sous pression et une inspiration du patient.

4. Procédé selon la revendication 3, dans lequel déterminer un degré de synchronisation comprend en outre comparer les probabilités combinées à une valeur seuil de bonne synchronisation.

5. Procédé selon l'une des revendications 1 à 4, dans lequel calculer une probabilité d'appui pour une trame vidéo comprend :

   détecter, dans la trame vidéo, des points représentatifs du doigt actuateur, et
   déterminer un mouvement relatif de descente du bout du doigt actuateur par rapport à une base du doigt, en comparaison d'au moins une trame vidéo temporellement précédente, la probabilité d'appui étant fonction de l'amplitude du mouvement de descente depuis une position de départ déterminée dans une trame vidéo précédente.

6. Procédé selon la revendication 5, dans lequel calculer une probabilité d'appui pour une trame vidéo comprend comparer l'amplitude du mouvement à une dimension de l'inhalateur-doseur sous pression dans la trame vidéo.

7. Procédé selon l'une des revendications 1 à 6, dans lequel calculer une probabilité de compression pour une trame vidéo comprend :
   comparer une longueur de l'inhalateur-doseur sous pression dans la trame vidéo avec une longueur référence de l'inhalateur-doseur sous pression.

8. Procédé selon l'une des revendications 1 à 7, dans lequel calculer une probabilité d'inhalation pour un segment audio comprend :

   convertir le segment audio en un spectrogramme, et
   utiliser le spectrogramme en entrée d'un réseau de neurones entrainé qui émet en sortie la probabilité d'inhalation.

9. Procédé selon l'une des revendications 1 à 8, dans lequel les étapes consistant à calculer les probabilités d'appui, de compression et d'inhalation sur des trames vidéo et segments audio postérieurs sont déclenchées par la détection d'un bon positionnement de l'inhalateur-doseur sous pression par rapport au patient dans des trames vidéos antérieures.

10. Procédé selon l'une des revendications 1 à 9, comprenant en outre une étape préalable de détermination d'une ouverture de l'inhalateur-doseur par détection d'un son clic caractéristique dans au moins un segment audio du signal audio, la détection mettant en œuvre un modèle de détection appris

11. Système informatique (100) comprenant un ou plusieurs processeurs (102) configurés pour :

obtenir un signal vidéo et un signal audio d'un patient utilisant un inhalateur-doseur sous pression (300),

calculer, pour chacune d'une pluralité de trames vidéo du signal vidéo, au moins l'une parmi une probabilité, dite d'appui, qu'un doigt actuateur du patient dans la trame vidéo soit en phase d'appui sur un élément déclencheur de l'inhalateur-doseur sous pression et une probabilité, dite de compression, que l'inhalateur-doseur sous pression dans la trame vidéo soit dans un état comprimé,

calculer, pour chacun d'une pluralité de segments audio du signal audio, une probabilité, dite d'inhalation, que le patient réalise, dans le segment audio, une inspiration combinée au flux aérosol,

déterminer un degré de synchronisation entre l'actionnement de l'inhalateur-doseur sous pression et une inspiration du patient à partir des probabilités d'appui, de compression et d'inhalation correspondant à des mêmes instants temporels, et

émettre en conséquence un signal de bon usage ou de mésusage de l'inhalateur-doseur sous pression au patient.

12. Support non-transitoire lisible par ordinateur stockant un programme qui, lorsqu'il est exécuté par un microprocesseur ou un système informatique, conduit le système à exécuter le procédé selon l'une des revendications 1 à 10.

Figure 1

Figure 2

M

I

310

300

320

**Figure 3**

P

p1(t)

p2(t)

p3(t)

s(t)

τ

→

S(t)

THR

t

T₀

**Figure 4**

Figure 4a

Démarrer enregistrement vidéo + audio — 500

Détecter visage — 505

Détecter et reconnaître inhalateur — 510

Vérifier doses — 515

Détecter ouverture — 520

Détecter expiration — 525

Détecter bon positionnement inhalateur — 530

Détecter synchronisation actionnement et inhalation — 535

Vérifier blocage respiration — 540

Détecter fermeture — 545

Fournir compte-rendu — 550

Figure 5

Europäisches
Patentamt

European
Patent Office

Office européen
des brevets

# RAPPORT DE RECHERCHE EUROPEENNE

Numéro de la demande

EP 22 16 6010

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | US 2013/063579 A1 (HANINA ADAM [US] ET AL) 14 mars 2013 (2013-03-14) * alinéas [0023], [0042], [0053], [0056], [0059], [0066]; revendications 1, 15; figures 2, 8 * * alinéas [0070], [0075], [0076] * ----- | 1-12 | INV. G16H20/13 G16H40/63 |

DOMAINES TECHNIQUES
RECHERCHES (IPC)

G16H

Le présent rapport a été établi pour toutes les revendications

| Lieu de la recherche | Date d'achèvement de la recherche | Examinateur |
|---|---|---|
| Munich | 20 juillet 2022 | Beligny, Samuel |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 22 16 6010

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-07-2022

| Document brevet cité au rapport de recherche | Date de publication | Membre(s) de la famille de brevet(s) | Date de publication |
|---|---|---|---|
| US 2013063579 A1 | 14-03-2013 | US 2013063579 A1 | 14-03-2013 |
| | | US 2018353052 A1 | 13-12-2018 |
| | | US 2020323417 A1 | 15-10-2020 |

EPO FORM P0460

Pour tout renseignement concernant cette annexe : voir Journal Officiel de l'Office européen des brevets, No.12/82

**RÉFÉRENCES CITÉES DANS LA DESCRIPTION**

**Documents brevets cités dans la description**

- US 2013063579 A

- WO 2019122315 A